Europäisches Patentamt

European Patent Office

Office européen des brevets

(11) Numéro de publication: **0 278 184**

**A1**

## (12) DEMANDE DE BREVET EUROPEEN

(21) Numéro de dépôt: **87400304.9**

(51) Int. Cl.⁴: **A61F 2/42**

(22) Date de dépôt: **11.02.87**

(43) Date de publication de la demande:
**17.08.88 Bulletin 88/33**

(84) Etats contractants désignés:
**AT BE CH DE ES FR GB GR IT LI LU NL SE**

(71) Demandeur: **Hermann, Thierry**
**Wenkenhaldenweg, 15**
**CH-4125 Richen(CH)**

Demandeur: **Pequignot, Michel**
**16 Villa Désiré Filleaud**
**F-92140 Clamart(FR)**

(72) Inventeur: **Hermann, Thierry**
**Wenkenhaldenweg, 15**
**CH-4125 Richen(CH)**
Inventeur: **Pequignot, Michel**
**16 Villa Désiré Filleaud**
**F-92140 Clamart(FR)**

(74) Mandataire: **CABINET BONNET-THIRION**
**95 Boulevard Beaumarchais**
**F-75003 Paris(FR)**

(54) Prothèse d'articulation, en particulier pour articulation de doigt.

(57) Il s'agit d'une prothèse du genre comportant deux broches (13, 14), avec intervenant entre celles-ci, et alignées suivant un axe transversal, pour l'une des broches, deux calottes (17A, 17B), et, pour l'autre, deux calottes (18A, 18B), qui, complémentaires des précédentes, sont en prise avec celles-ci.

Suivant l'invention, les calottes (17A, 17B) de la broche (13) appartiennent chacune respectivement à deux pions (20A, 20B) mobiles transversalement par rapport à l'ensemble.

Application, notamment, aux prothèses d'articulation pour doigts.

EP 0 278 184 A1

FIG.2

## "Prothèse d'articulation, en particulier pour articulation de doigt"

La présente invention concerne d'une manière générale les prothèses d'articulation, et elle vise plus particulièrement celles suivant lesquelles seule une articulation autour d'un axe doit être autorisée.

C'est le cas, par exemple, des articulations intervenant entre les phalanges successives d'un doigt, qui, ainsi qu'on le sait, constituent des diarthroses trochléennes, c'est-à-dire des jointures articulaires dans lesquelles un os roule sur un autre à la faveur d'une gorge, formant poulie, que présente à cet effet celui-ci.

Les prothèses actuellement proposées en substitution à une telle articulation en cas de traumatismes ne donnent pas totalement satisfaction.

Il y a tout d'abord des prothèses en matière souple, caoutchouc naturel ou synthétique par exemple, dont la tenue mécanique s'avère parfois insuffisante.

Il y a ensuite, des prothèses mécaniques comportant, par exemple, deux broches, destinées à être engagées, chacune respectivement, dans les deux os à relier, et, intervenant entre lesdites broches, une simple chape, solidaire de l'une de celles-ci, et un axe, solidaire de l'autre.

De telles prothèses mécaniques présentent un double inconvénient.

Tout d'abord, elles ne se prêtent pas, sans cassure ou complication de structure rédhibitoire, à une quelconque possibilité de luxation, ou autrement dit de déboîtement, alors même qu'au contraire une telle possibilité doit physiologiquement être recherchée.

En outre, les surfaces articulaires qu'elles mettent en jeu étant des surfaces ouvertes, c'est-à-dire des surfaces débouchant latéralement en plusieurs endroits, puisqu'il s'agit de surfaces cylindriques successives, elles peuvent, par invasion osseuse, être progressivement l'objet d'un grippage.

Il a cependant également été déjà proposé des prothèses dans lesquelles les surfaces articulaires sont avantageusement des surfaces internes, qui, ne débouchant pas latéralement à l'extérieur, se trouvent ainsi dûment protégées.

C'est le cas par exemple dans le brevet français No 1.484.143 et dans le brevet américain No. 4.059.854.

En pratique dans les prothèses d'articulation correspondantes, qui, comme précédemment, sont des prothèses équipées de broches pour leur implantation, les surfaces articulaires comportent, pour l'une des broches, dite ci-après par simple commodité broche porteuse, deux calottes, plus ou moins sphériques ou sphéroïdes, qui, centrées l'une et l'autre sur un axe perpendiculaire à ladite broche porteuse, en étant orientées en sens opposés l'une par rapport à l'autre, sont portées par ladite broche porteuse, et, pour l'autre desdites proches, dite ci-après par simple commodité broche portée, deux autres calottes plus ou moins sphériques ou sphéroïdes, qui, complémentaires chacune respectivement des précédentes, et propres ainsi à être en prise avec celles-ci, sont portées par ladite broche portée.

Mais, du fait même du caractère interne des surfaces articulaires ainsi constituées, il faut, dans l'un et l'autre cas, lors de la mise en place de la prothèse, procéder à un engagement à force de l'une des broches par rapport à l'autre.

Outre qu'un tel engagement à force n'est pas nécessairement aisé à assurer, il implique inévitablement que la partie concernée de l'une au moins des broches, celle formant alors un élément femelle par rapport à l'autre, présente une certaine capacité de déformation, au détriment éventuel de la tenue ultérieure de l'ensemble.

Dans le brevet français qui, déposé sous le No 66 19625, a été publié sous le No 2.356.406, il est par ailleurs également proposé une prothèse d'articulation dont l'un des constituants a pour surfaces articulaires des calottes plus ou moins sphériques ou sphéroïdes.

Mais, les surfaces articulaires de l'autre de ces constituants sont orientées vers l'extérieur, au risque d'un envahissement osseux ultérieur, et, pour la venue en prise des surfaces articulaires du premier, elles se trouvent recoupées par des rainures, propres à accentuer encore un tel risque. De plus, il s'agit, dans ce brevet français No 77 19625, d'une prothèse dont les constituants sont dépourvus de broche, leur implantation sur les os à relier se faisant à l'aide de vis, et, de surcroît, d'une prothèse qui est de réalisation relativement complexe, et donc coûteuse, et dont les possibilités de luxation apparaissent incertaines.

La présente invention a d'une manière générale pour objet une disposition permettant d'obvier à ces inconvénients.

De manière plus précise, elle a pour objet une prothèse d'articulation du genre comportant deux broches destinées à être engagées, chacune respectivement, directement ou indirectement, dans les deux os à relier, et, intervenant entre lesdites broches, des surfaces articulaires, lesdites surfaces articulaires comportant, pour l'une des broches, dite ici par simple commodité broche porteuse, deux calottes, qui, centrées l'une et l'autre sur un axe perpendiculaire à ladite broche porteuse en étant orientées en sens opposés l'une par rapport à l'autre, sont portées par ladite broche porteuse, et, pour l'autre desdites broches, dite ici par simple commodité broche portée, deux autres calottes, qui, complémentaires chacune respectivement des précédentes, et propres ainsi à être en prise avec celles-ci, sont portées par ladite broche portée, cette prothèse d'articulation étant d'une manière générale caractérisée en ce que les calottes de la broche porteuse sont chacune respectivement portées par deux pions qui sont montés mobiles transversalement par rapport aux deux broches, ladite broche porteuse comportant deux bras présentant chacun respectivement, dans l'alignement l'un de l'autre, duex logements dans lesquels sont engagés à coulissement lesdits pions.

Du fait de leur mobilité, les pions ainsi mis en oeuvre suivant l'invention peuvent être déplacés d'une position relative écartée d'attente, pour laquelle ils n'interfèrent en rien avec le trajet qui doit être celui de la broche portée lors de son engagement relatif avec la broche porteuse, à une position relative rapprochée de service, pour laquelle, cet engagement étant effectué, ils sont en prise, par les calottes qu'ils portent, avec les calottes de cette broche portée.

Ainsi, l'engagement relatif de la broche portée avec la broche porteuse n'implique avantageusement la déformation élastique d'aucun des constituants essentiels de l'une ou l'autre de ces broches, ce qui est favorable au maintien ultérieur d'un bonne orientation de ces broches l'une par rapport à l'autre.

En outre, grâce à la disposition suivant l'invention, les surfaces articulaires qui interviennent entre ces broches sont avantageusement des surfaces internes dont aucune ne débouche directement à l'extérieur, et le risque qu'elles soient intempestivement l'objet d'une quelconque invasion osseuse est avantageusement limitè.

En pratique, les logements dans lesquels sont montés mobiles les pions mis en oeuvre à cet effet suivant l'invention débouchent de préférence à l'extérieur, en sorte que, pour leur passage de leur position reculée d'attente à leur position rapprochée de service, il est très simplement possible d'agir de l'extérieur sur ces pions.

La mise en oeuvre de la prothèse d'articulation suivant l'invention s'en trouve dès lors très aisée : l'engagement relatif de la broche portée avec la broche porteuse ayant été effectué alors que les pions sont en position écartée d'attente, il suffit de faire passer ceux-ci en position rapprochée de service en agissant pour ce faire d'une seule main sur l'ensemble.

En outre, du fait de la capacité de déplacement des pions qui les portent les calottes de la broche porteuse peuvent, si désiré, jouer par rapport aux calottes de la broche portée avec lesquelles elles sont en prise, et il peut ainsi exister, si désiré, de manière très simple et sûre, et sand risque de casse, une possibilité de luxation.

Mais, conjointement, suivant une forme de réalisation de l'invention, il est associé, à l'une au moins des broches, et, de préférence, à chacune de celles-ci, un fourreau, dans lequel est engagée, par une queue, une telle broche, et par rapport auquel elle peut jouer longitudinalement.

C'es alors ce fourreau qui est scellé, ancré, ou vissé, dans la cavité osseuse correspondante.

Dès lors, le jeu possible entre un tel fourreau et la broche correspondante favorise avantageusement une possibilité de luxation à ce niveau, conjointement à la possibilité précédente ou en substitution à celle-ci.

Les caractéristiques et avantages de l'invention ressortiront d'ailleurs de la description qui va suivre, à titre d'exemple, en référence aux dessins - schématiques annexés sur lesquels :

la figure 1 est une vue en coupe axiale d'une prothèse d'articulation suivant l'invention, en place entre les deux phalanges d'un doigt ;

la figure 2 est à échelle supérieure, une vue en coupe axiale de cette seule prothèse d'articulation, représentée isolément ;

la figure 3 reprend, à échelle supérieure, le détail de la figure 2 repéré par un encart III sur celle-ci ;

la figure 4 est une vue latérale de cette prothèse d'articulation, suivant la flèche IV de la figure 2 ;

la figure 5 en est une vue en coupe transversale, suivant la ligne V-V de la figure 4 ;

les figures 6A, 6B sont des vues en coup axiale qui, analogues à celle de la figure 2, illustrent deux phases successives de mise en oeuvre de la prothèse d'articulation suivant l'invention ;

les figures 7, 8 sont des vues respectivement analogues à celles des figures 2, 3, pour une variante de réalisation de la prothèse d'articulation suivant l'invention ;

la figure 9 est une vue analogue à celle de la figure 6A, pour cette variante de réalisation ;

la figure 10 est, suivant la flèche X de la figure 9, une vue latérale d'une des broches que comporte cette variante de réalisation ;

la figure 11 est une vue en coupe axiale qui, reprenant pour partie celle de la figure 7, se rapporte à une variante de réalisation d'un des constituants de la prothèse d'articulation suivant l'invention ;

la figure 12 est une vue en coupe axiale, qui, reprenant elle aussi pour partie celle de la figure 7, se rapporte à une autre variante de réalisation de la prothèse d'articulation suivant l'invention ;

la figure 13 est une vue en élévation du fourreau que comporte éventuellement une telle prothèse d'articulation ;

la figure 14 est une vue en plan de ce fourreau, suivant la flèche XI de la figure 10.

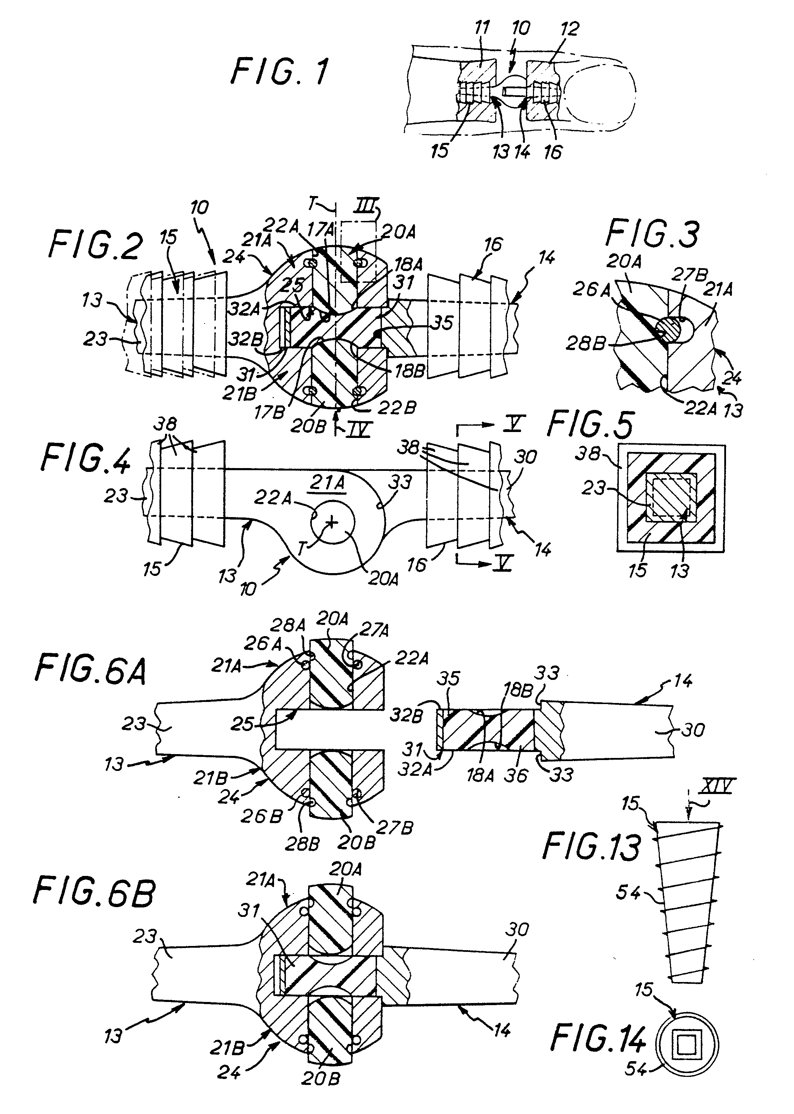

Tel qu'illustré à la figure 1, la prothèse d'articulation 10 suivant l'invention est destinée à relier deux os 11, 12, en l'espèce les deux phalanges d'un doigt.

Elle comporte pour ce faire deux broches 13, 14, qui, soit directement, soit indirectement, par l'intermédiare, tel que représenté, et ainsi qu'il sera décrit plus en détail ultérieurement, de fourreaux 15, 16, sont destinées à être engagées, chacune respectivement, dans les os 11, 12 à relier, et entre lesquelles interviennent des surfaces articulaires, ces surfaces articulaires comportant, pour l'une d'elles, la broche 13, dite ici par simple commodité broche porteuse, deux callotes 17A, 17B, globalement sphériques ou sphéroïdes, qui, portées par ladite broche porteuse 13, sont orientées en sens opposée l'une par rapport à l'autre, et, pour l'autre, la broche 14, dite ici par simple commodité broche portée, deux autres calottes 18A, 18B, qui, complémentaires chacune respectivement des précédentes, et propres ainsi à être en prise avec celles-ci, sont portées par ladite broche portée 14, en coopération avec, tel que précisé plus en détail ultérieurement, des moyens, dits ici par simple commodité moyens de restriction, propres à n'autoriser qu'un pivotement relatif autour d'un axe transversal T des calottes 18A, 18B de la broche portée 14 par rapport aux calottes 17A, 17B de la broche porteuse 13.

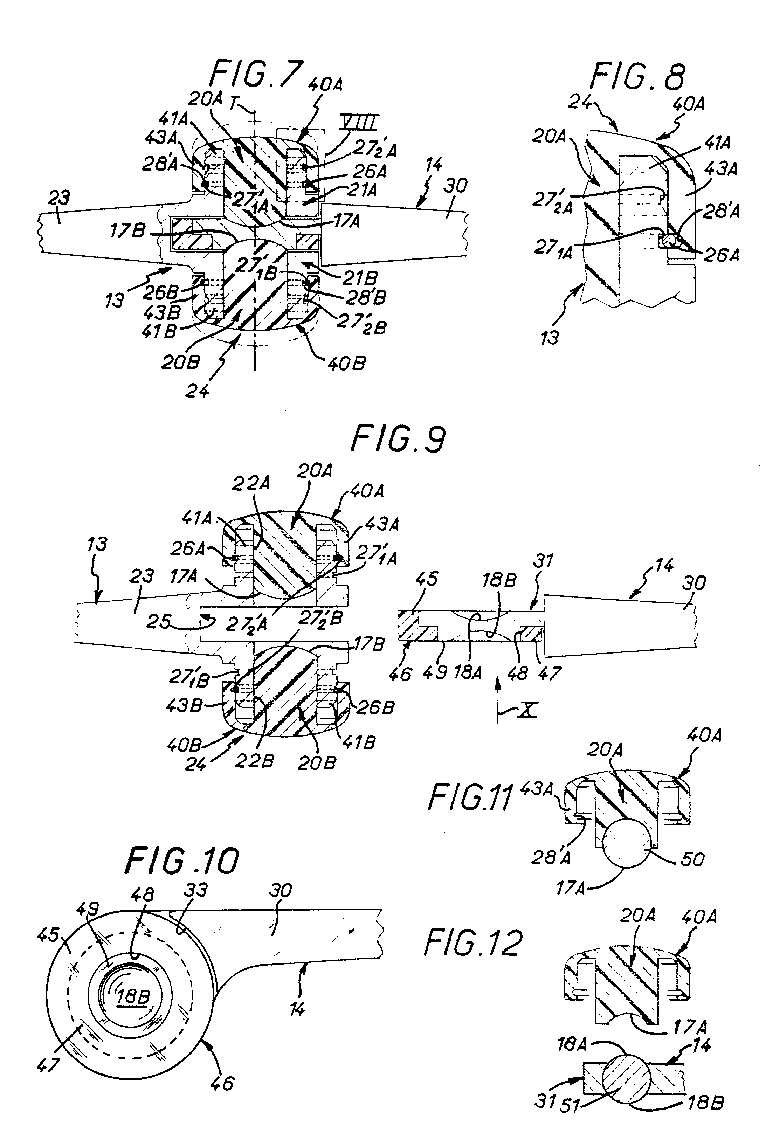

Cet axe transversal T qui s'étend sensiblement perpendiculairement aux broches 13, 14, et donc sensiblement perpendiculairement aux os 11, 12 concernés, a été représenté en traits interrompus sur les figures 2 et 7 ; il est en outre schématisé par sa trace sur la figure 4.

En pratique, les calottes 17A, 17B, d'une part, et 18A, 18B, d'autre part, sont des calottes sphériques, toutes centrées sur l'axe transversal T.

Tel que représenté, elles s'étendent chacune sur un angle solide qui, par exemple, est inférieur à 90°.

Mais il va de soi que, en variante, cet angle solide peut être supérieur à 90°.

Dans les formes de réalisation représentées sur les figures 1 à 11, les calottes 17A, 17B de la broche porteuse 13 sont convexes, et, en correspondance, les calottes 18A, 18B de la broche portée sont concaves.

Mais, en variante, et c'est le cas, tel qu'il sera décrit plus en détail ultérieurement, pour la forme de réalisation représentée à la figure 12, une disposition inverse peut être adoptée.

Quoi qu'il en soit, et suivant l'invention, les calottes 17A, 17B de la broche porteuse 13 sont chacune respectivement portées par deux pions 20A, 20B qui sont montés mobiles transversalement par rapport aux broches 13, 14, suivant, en pratique, l'axe transveral T, ladite broche porteuse 13 comportant à cet effet deux bras 21A, 21B présentant chacun respectivement, dans l'alignement l'un de l'autre, suivant ledit axe transversal T, deux logements 22A, 22B dans lesquels sont engagés à coulissement lesdits pions 20A, 20B.

En pratique, ces logements 22A, 22B, qui sont des logements cylindriques de section transversale circulaire, débouchent à l'extérieur, à la surface des bras 21A, 21B, sur les faces opposées de ceux-ci, en sorte qu'il est possible d'agir de l'extérieur sur les pions 20A, 20B correspondants.

Dans la forme de réalisation plus particulièrement représentée sur les figures 1 à 6, la broche porteuse 13 est massive, et elle est par exemple réalisée en métal.

Elle comporte, d'un seul tenant, une queue en métal 23, par laquelle elle est adaptée à être engagée dans l'os concerné, en pratique par l'intermédiaire du fourreau 15, et qui, dans la forme de réalisation représentée, a, extérieurement, un contour pyramidal, à section transversale quadrangulaire, et une tête 24, qui, formée des bras 21A, 21B, a, extérieurement, un contour globalement sphéroïde, avec, cependant, la tranche de ces bras 21A, 21B arasée suivant une surface cylindrique de contour circulaire centré sur l'axe transversal T.

Les bras 21A, 21B de cette broche porteuse 13 délimitent entre eux, perpendiculairement à l'axe transversal T, une fente 25, dont les faces opposées sont parallèles l'une à l'autre, et dans laquelle débouchent les logements 22A, 22B correspondants.

Dans la forme de réalisation représentée sur les figures 1 à 6, les pions 20A, 20B sont massifs, et il s'agit par exemple de pions en matière synthétique choisie pour être biologiquement compatibles avec les tissus osseux.

A leur extrémité interne, ils forment, par eux-mêmes, les calottes 17A, 17B correspondantes.

Quant à leur extrémité externe, elle est globalement arrondie de manière à se fondre, en service, avec la surface extérieure des bras 21A, 21B, en continuité avec celle-ci.

De préférence, et tel que représenté, entre ces pions 20A, 20B et les bras 21A, 21B qui les portent sont prévus des moyens d'encliquetage propres à un blocage en position de ces pions 20A, 20B dans leurs logements 22A, 22B respectifs.

Par exemple, et tel que représenté, il est prévu, pour chacun de ces pions 20A, 20B, entre cuir et chair, un anneau élastiquement déformable 26A, 26B, par exemple un anneau affecté localement d'une fente radiale, qui, disposé dans une gorge 27A, 27B du bras 21A, 21B concerné susceptible de l'absorber complètement, figures 6A, 6B, est apte à coopérer, conjointement, avec une gorge 28A, 27B d'un tel pion 20A, 20B, propre, elle, à n'en permettre qu'un engagement partiel, figures 2, 3.

Comme la broche porteuse 13, la broche portée 14 comporte, d'un seul tenant, une queue 30, qui a extérieurement un contour globalement pyramidal de section transversale quadrangulaire, et une tête qui se réduit à un simple flasque 31.

Sur les faces latérales 32A, 32B de ce flasque 31, qui sont planes, et qui s'étendent globalement parallèlement l'une à l'autre, sensiblement perpendiculairement à l'axe transversal T, s'étendent chacune respectivement, en creux, les calottes 18A, 18B correspondantes.

En pratique, dans la forme de réalisation représentée, les faces latérales 32A, 32B du flasque 31 sont légèrement en retrait par rapport aux faces correspondantes de la queue 30 associée, et les épaulements 33 que présente de ce fait la broche porteuse 14 à la racine de ce flasque 31 ont, en plan, un contour circulaire complémentaire de celui de la tranche des bras 21A, 21B de la broche porteuse 13.

Quoi qu'il en soit, l'épaisseur du flasque 31 de la broche portée 14 est sensiblement égale à la largeur de la fente 25 de cette broche proteuse 13, tout en étant légèrement inférieure à celle-ci, de manière à ce que ce flasque 31 puisse être inséré dans cette fente 25.

En plan, le flasque 31 de la broche portée 14 a extérieurement un contour circulaire, à l'image de celui de la tête 24 de la broche porteuse 13, pour se fondre dans celui-ci.

Dans la forme de réalisation représentée, ce flasque 31 est largement évidé d'une ouverture 35 et il forme ainsi un anneau dans lequel est enchâssée une pièce 36 formant les calottes 18A, 18B correspondantes.

En pratique, cette pièce 36 est, dans cette forme de réalisation, une plaquette, dont les faces, qui sont chacune respectivement à niveau avec celles du flasque 31 qu'elle équipé, présentent, en creux, dos à dos, les calottes 18A, 18B.

Comme précédemment, cette plaquette est de préférence réalisée en matière synthétique biologiquement compatible avec les tissus osseux.

Enfin, dans la forme de réalisation représentée, et comme déjà indiqué ci-dessus, il est associé aux broches 13, 14, et plus précisément aux queues 23, 30 de celles-ci, des fourreaux 15, 16, dans lesquels, par lesdites queues 23, 30, sont engagées lesdites broches 13, 14, et par rapport auxquels celles-ci peuvent jouer longitudinalement.

Comme également déjà indiqué ci-dessus, les queues 23, 30 des broches 13, 14 ont, extérieurement, un contour pyramidal, en étant très légèrement effilées en direction de leur extrémité libre.

Corollairement, les fourreaux 15, 16 ont, intérieurement, un contour pyramidal complémentaire du précédent.

Les emmanchements correspondants sont donc, dans ce cas, des emmanchements pyramidaux.

Si, dans la forme de réalisation représentée, les sections transversales correspondantes sont quadrangulaires, ces sections transversales pourraient tout aussi bien être triangulaires.

De manière plus générale, elles sont de préférence polygonales, pour éviter normalement toute rotation des broches 13, 14 par rapport à leurs fourreaux 15, 16.

Dans la forme de réalisation représentée, les fourreaux 15, 16 ainsi mis en oeuvre ont également un contour extérieur pyramidal, à l'image de leur contour intérieur.

En outre, sur une portion au moins de leur longueur, ils présentent transversalement, des crans 38.

Ces fourreaux 15, 16 sont ainsi destinés à être engagés à force dans les os 11, 12 à relier, après excavation convenable du ceux-ci et arasement de leur extrémité concernée.

Par leurs crans 38, ils viennent alors s'ancrer dans ces os 11, 12.

Quoi qu'il en soit, une fois les fourreaux 15, 16 en place dans les os 11, 12 à relier, il est procédé à l'engagement des broches 13, 14 dans ces fourreaux 15, 16 par leur queue 23, 30.

Les pions 22A, 22B de la broche porteuse 13 étant en position saillante par rapport aux bras 21A, 21B de celle-ci, et donc en position écartée l'un par rapport à l'autre, avec les anneaux élastiquement déformables 26A, 26B correspondants élastiquement refoulés dans les gorges 27A, 27B de ces bras 21A, 21B, figure 6A, la broche portée 14 est alors engagée, par son flasque 31, dans la fente 25 ainsi dégagée de la broche porteuse 13, jusqu'à contact de ses épaulements circulaires 41 avec la tranche correspondante desdits bras 21A, 21B, figure 6B.

Les calottes 18A, 18B du flasque 31 de la broche portée 14 se trouvent alors dans l'alignement des pions 20A, 20B de la brcohe porteuse 13.

Il suffit, dès lors, de refouler dans leurs logements 22A, 22B ces pions 20A, 20B, ou autrement dit, de les rapprocher l'un de l'autre, jusqu'à ce que la calotte 17A, 17B formant leur extrémité interne vienne en prise avec la calotte 18A, 18B correspondante du flasque 31 de la broche portée 14.

Au terme de cette opération, l'extrémité extérieure des pions 20A, 20B se trouve avantageusement en continuité avec la surface arrondie correspondante des bras 21A, 21B qu'ils équipent, et, conjointement, les anneaux élastiquement déformables 26A, 26B s'enclenchent élastiquement dans les gorges 28A, 28B de ces pions 20A, 20B, ce qui se traduit, avantageusement, par un "clic" sonore matérialisant la bonne exécution du montage à assurer.

Il est procédé ensuite, de manière usuelle, à la liguration des tendons concernés.

Ainsi qu'on le comprendra, du fait que les calottes 18A, 18B de la broche portée 14 appartiennent chacune respectivement à des surfaces distinctes, le seul mouvement naturellement possible entre cette broche portée 14 et la broche porteuse 13 est un mouvement de rotation autour de l'axe transversal T sur lequel sont l'une et l'autre centrées ces calottes 18A, 18B.

Ainsi se trouvent très simplement réalisée les moyens de "restriction" mentionnés ci-dessus.

Dans la forme de réalisation représenté, ceux-ci se trouvent par ailleurs confirmés par le fait que la broche portée 14 est conjointement engagée par un flasque 31 dans la broche porteuse 13, avec, cependant, un jeu de montage plus ou moins grand.

Sur les figures 1 et 6B relatives à cette forme de réalisation, ce jeu de montage a été supposé nul.

Mais il a été dûment représenté sur la figure 7 relative à la forme de réalisation qui sera décrite ultérieurement.

Quoi qu'il en soit, lorsque, comme décrit, les pions 20A, 20B mis en oeuvre suivant l'invention sont engagés, par leurs calottes 17A, 17B, dans les calottes 18A, 18B de la broche portée 14, ils maintiennent normalement celle-ci en prise avec la broche porteuse 13.

Mais ainsi qu'on le comprendra également, il existe, cependant, de manière très simple et sûre, et sans risque de casse, avec la prothèse d'articulation suivant l'invention, une possibilite de luxation.

Préférentiellement, si une luxation se produit, elle intervient entre l'une au moins des broches 13, 14 et le fourreau 15, 16 correspondant, par un déplacement longitudinal d'une telle broche 13, 14 par rapport à ces fourreaux 15, 16.

Du fait que, comme indiqué ci-dessus, l'emmanchement de la queue 23, 30 d'une telle broche 13, 14 avec son fourreau 15, 16 est avantageusement pyramidal, il n'y a pas normalement de possibilité de rotation entre elle et ce fourreau.

Mais, en cas de luxation, il se développe au contraire avantageusement une telle possibilité de rotation, un jeu apparaissant alors entre la queue 23, 30 de la broche 13, 14 concernée et son fourreau 15, 16, du fait de son déplacement longitudinal par rapport à celui-ci.

Cette possibilité de rotation, qui reste limitée, peut avantageusement permettre que l'effort de torsion relatif appliqué aux phalanges concernées ne conduise de manière intempestive à un descellement de la prothèse.

Le rappel entre les phalanges en cause se fait ensuite de manière naturelle par les tendons intervenant usuellement entre elles.

Bien entendu, les fourreau 15, 16 sont de préférence en matière synthétique biologiquement compatible avec les tissus osseux.

Les crans 38 qu'ils comportent, et qui interviennent à la manière de barbillons, peuvent suffire à leur implantation dans les cavités osseuses correspondantes.

La matière de celles-ci étant en pratique une matière spongieuse très évolutive, les intervalles entre ces crans 38 sont très rapidement l'objet d'un envahissement osseux important consolidant leur fixation dans les phalanges concernées.

Mais, en variante, il peut être procédé à un scellement au ciment de ces fourreaux 15, 16.

Dans la variante de réalisation représentée sur les figures 7 à 10, les pions 20A, 20B de la broche porteuse 13 sont chacun respectivement solidaires d'une coiffe 40A, 40B par laquelle ils recouvrent au moins pour partie les bras 21A, 21B de cette broche porteuse 13.

En pratique, chacun de ces bras 21A, 21B forme alors un bossage annulaire 41A, 41B, dans lequel s'étend le logement 22A, 22B correspondant, et qui est donc en forme de puits, et, conjointement, la coiffe 40A, 40B associée comporte une jupe 43A, 43B par laquelle elle entoure, sur une partie au moins de sa hauteur, ce bossage annulaire 41A, 41B.

Une telle coiffe 40A, 40B présente divers avantages.

Tout d'abord, il est possible, tel que - schématisé en traits interrompus sur la figure 7, de lui donner, suivant l'axe transversal T, des épaisseurs différentes.

Ainsi, pour une même prothèse d'articulation c'est-à-dire pour une prothèse d'articulation constituée des mêmes broches 13, 14, il est possible de disposer de coiffes 40A, 40B différentes. et de choisir parmi celles-ci celles dont l'épaisseur est la mieux adaptée à la grosseur du doigt à traiter.

La mise en oeuvre d'une coiffe 40A, 40B permet en outre avantageusement d'étendre sur un diamètre supérieur les moyens d'encliquetage prévus pour le blocage en position des pions 20A, 20B.

En effet, dans la forme de réalisation représentée sur les figures 7 à 10, ces moyens d'encliquetage interviennent, non plus directement, entre ces pions 20A, 20B et leurs logements 22A, 22B, mais entre la jupe 43A, 43B de la coiffe 40A, 40B dont ils sont solidaires et le bossage annulaire 41a, 41B du bras 21A, 21B correspondant.

Du fait que ces moyens d'encliquetage s'étendent ainsi sur un diamètre supérieur à celui des seuls pions 20A, 20B, leur réalisation s'en trouve facilitée, et leur fonctionnement amélioré.

En pratique, dans la forme de réalisation représentée sur les figures 7 à 10, ces moyens d'encliquetage, qui, comme précédemment, comportant un anneau élastiquement déformable 26A, 26B, interviennent à deux niveaux différents.

En effet, alors que, sur la surface interne de la jupe 43A, 43B des coiffes 40A, 40B, il n'est prévu, au voisinage de l'extrémité libre de celle-ci, qu'une seule gorge 28'A, 28'B propre à coopérer avec un tel anneau élastiquement déformable 26A, 26B, il est prévu, échelonnées axialement en hauteur le long de l'axe transversal T, sur la surface extérieure des bossages annulaires 41A, 41B correspondants, deux gorges 27'₁A, 27'₁B-27'₂A, 27'₂B.

Comme précédemment, les flancs de la gorge 27'₁A, 27'₁B sont droits, en s'étendant sensiblement perpendiculairement à l'axe transversal T, et cette gorge 27'₁A, 27'₁B est établie à un niveau correspondant à la venue en prise des pions 20A, 20B avec les calottes 18A, 18B de la broche portée 14.

Par contre, la gorge 27'₂A, 27'₂B est établie à un niveau pour lequel la calotte 17A, 17B des pions 20A, 20B de la broche porteuse 13 est largement en retrait par rapport aux faces correspondantes de la fente 25 de celle-ci.

Tel qu'illustré à la figure 9, elle assure un encliquetage en position écartée d'attente des pions 20A, 20B propre à maintenir en toute sûreté ceux-ci à l'écart de la fente 25, et donc à permettre l'insertion, dans cette fente 25 du flasque 31 de la broche portée 14.

De préférence, et tel que représenté, pour faciliter le caractère débrayable de cette position initiale écartée d'attente, celui des flancs de cette gorgfe 27'₂A, 27'₂B qui est le plus proche de la fente 25 est oblique par rapport à l'axe transversal T.

Par ailleurs, dans la forme de réalisation représentée sur les figures 7 à 10, le flasque 31 de la broche portée 14 est massif, et il forme donc par lui-même les calottes 18A, 18B correspondantes.

En outre, dans cette forme de réalisation, ce flasque 31 est gainé, sur une partie au moins de son pourtour, par un bandage en matière souple 45, propre à ménager le tendon extenseur du doigt concerné, en lui évitant de frotter sur une partie métallique.

En pratique, ce bandage 45 appartient à une pièce 46 qui, transversalement, forme un flasque 47 par une ouverture centrale 48 duquel cette pièce 46 est engagée sur un bossage 49 du flasque 31, ce bossage 49 résultant par exemple, et tel que représenté, d'une simple réduction locale annulaire de l'épaisseur de ce flasque 31.

Dans la variante de réalisation illustrée par la figure 11, à l'extrémité de chacun des pions 20A, 20B est enchâssée une pièce 50 formant la calotte 17A, 17B correspondante.

Pour plus de simplicité, seul l'un des pions 20A, 20B, et il s'agit en l'espèce du pion 20A, a été représenté sur cette figure 11.

En outre, il s'agit, dans la forme de réalisation représentée, d'un pion 20A dont est solidaire une coiffe 40A.

Mais, bien entendu, la variante de réalisation en cause pourrait tout aussi bien concerner la forme de réalisation d'un tel pion illustré sur les figures 1 à 6.

Quoi qu'il en soit, dans la forme de réalisation représentée, la pièce 50 est une bille.

Par exemple, il peut s'agir d'une bille en alumine, saphir ou céramique, voire même d'une bille en métal.

Dans ce qui précède, les calottes 17A, 17B de la broche porteuse 13 sont convexes, et les calottes 18A, 18B correspondantes de la broche portée 14 sont concaves.

Tel qu'illustré sur la figure 12, où, par mesure de simplicité, seule l'une des calottes de la broche porteuse 13, en l'espèce la calotte 17A, a été représentée, une disposition inverse peut être adoptée, les calottes 17A, 17B de cette broche porteuse 13 étant alors concaves et celles 18A, 18B correspondantes de la broche portée 14 convexes.

Dans la forme de réalisation représentée, les calottes 18A, 18B de la broche portée 14 peuvent alors par exemple appartenir conjointement à une même pièce 51 enchâssée dans le flasque 31 de cette broche portée 14.

Par exemple et tel que représenté, cette pièce 51 peut, comme précédemment, être une bille.

Dans un tel cas, les moyens de "restriction" sont exclusivement assurés par le flasque 31 de la broche portée 14 en coopération avec les bras 21A, 21B de la broche porteuse 13 qui l'enserrent.

Dans la variante de réalisation - schématiquement représentée sur les figures 13 et 14 pour l'un d'eux, en l'espèce le fourreau 15, l'un et/ou l'autre des fourreaux 15, 16 préférentiellement associés aux broches 13, 14 de la prothèse d'articulation 10 suivant l'inventin peut être rapporté par vissage dans les os 11, 12 à relier, ces fourreaux présentant alors extérieurement un filet 54, qui peut soit faire corps avec eux, soit résulter d'une pièce rapportée sur eux.

Une telle disposition a pour avantage de permettre de rectifier par rotation la position des fourreaux 15, 16 dans les os 11, 12 concernés avant l'engagement dans ces fourreaux 15, 16 de la queue 23, 30 des broches 13, 14 correspondants.

Bien entendu, la présente invention ne se limite pas aux formes de réalisation décrites et représentées, mais englobe toute variante d'exécution et/ou de combinaison de leurs divers éléments.

En particulier, si, dans ce qui précède, il a été de préférence associé aux broches de la prothèse d'articulation suivant l'invention des fourreaux propres notamment à conférer à celle-ci une possibilité de luxation, il subsiste avantageusement, par les moyens d'encliquetage mis en oeuvre par ailleurs, une telle possibilité de luxation lorsque de tels fourreaux ne sont pas utilisés.

Il suffit d'adapter en conséquence ces moyens d'encliquetage, de manière à ce que le blocage en position qu'ils ont à charge d'assurer pour les pions concernés soit débrayable.

Par exemple, lorsque ces moyens d'encliquetage comportent un anneau élastiquement déformable, les flancs des gorges correspondantes peuvent être plus ou moins obliques, comme cela a été décrit pour l'un d'eux pour la forme de réalisation dans laquelle il est prévu que de tels moyens d'encliquetage s'étendent à deux niveaux distincts.

## Revendications

1. Prothèse d'articulation, du genre comportant deux broches (13, 14) destinées à être engagées, chacune respectivement, directement ou indirectement, dans les deux os à relier, et, intervenant entre lesdites broches (13, 14), des surfaces articulaires, lesdites surfaces articulaires comportant, pour l'une (13) des broches, dites ici par simple commodité broche porteuse, deux calottes (17A, 17B) qui, portées par ladite broche porteuse (13), sont orientées en sens opposés l'une par rapport à l'autre, et, pour l'autre (14) desdites broches, dite ici par simple commodité broche portée, deux autres calottes (18A, 18B), qui, complémentaires chacune respectivement des précédentes et propres ainsi à être en prise avec celles-ci, sont portées par ladite broche portée (14), caractérisée en ce que les calottes (17A, 17B) de la broche porteuse (13) sont chacune respectivement portées par deux pions (20A, 20B) qui sont montés mobiles transversalement par rapport aux deux broches (13, 14), ladite broche porteuse (13) comportant deux bras (21A, 21B) présentant chacun respectivement, dans l'alignement l'un de l'autre, deux logements (22A, 22B) dans lesquels sont engagés à coulissement lesdits pions (20A, 20B).

2. Prothèse d'articulation suivant la revendication 1, caractérisée en ce que lesdits logements (22A, 22B) débouchent à l'extérieur, en sorte qu'il est possible d'agir de l'extérieur sur lesdits pions (20A, 20B).

3. Prothèse d'articulation suivant la revendication 2, caractérisée en ce que lesdits pions (20A, 20B) sont chacun respectivement solidaires d'une coiffe (40A, 40B) par laquelle ils recouvrent au moins pour partie les bras (21A, 21B) correspondants.

4. Prothèse d'articulation suivant la revendication 3, caractérisée en ce que chacun des bras (21A, 21B) forme un bossage annulaire (41A, 41B), dans lequel s'étend le logement (22A, 22B) correspondant, et la coiffe (40A, 40B) du pion (20A, 20B) associé comporte une jupe (43A, 43B) par laquelle elle entoure ledit bossage annulaire (41A, 41B).

5. Prothèse d'articulation suivant l'une quelconque des revendications 1 à 4, caractérisée en ce que, entre les pions (20A, 20B) et les bras (21A, 21B) qui les portent sont prévus des moyens d'encliquetage propres à un blocage en position desdits pions (20A, 20B) dans leurs logements (22A, 22B).

6. Prothèse d'articulation suivant la revendication 5, caractérisée en ce qu'il est prévu des moyens d'encliquetage à deux niveaux différents.

7. Prothèse d'articulation suivant, prises conjointement, d'une part, la revendication 4, et, d'autre part, l'une quelconque des revendications 5, 6, caractérisée en ce que, les moyens d'encliquetage interviennent entre le bossage annulaire (41A, 41B) des bras (21A, 21B) et la jupe (43A, 43B) de la coiffe (40A, 40B) correspondante.

8. Prothèse d'articulation suivant l'une quelconque des revendications 1 à 7, caractérisée en ce que les bras (21A, 21B) de la broche porteuse (13) enserrent un flasque (31) de la broche portée (14), sur les facews (32A, 32B) duquel s'étendent chacune respectivement les calottes (18A, 18B) de celle-ci.

9. Prothèse d'articulation suivant la revendication 8, caractérisée en ce que le flasque (31) de la broche portée (14) forme un anneau dans lequel est enchâssée une pièce (36, 51) formant les calottes (18A, 18B) correspondantes.

10. Prothèse d'articulation suivant la revendication 8, caractérisée en ce que le flasque (31) de la broche portée (14) est massif et forme par lui-même les calottes (18A, 18B) correspondantes.

11. Prothèse d'articulation suivant l'une quelconque des revendications 8 à 10, caractérisée en ce que, sur une partie au moins de sons pourtour, le flasque (31) de la broche portée est gainé par un bandage (45) en matière souple.

12. Prothèse d'articulation suivant la revendication 11, caractérisée en ce que ledit bandage (45) appartient à une pièce (46) qui, transversalement, forme un flasque (47) par une ouverture centrale (48) duquel elle est engagée sur un bossage (49) du flasque (31) de la broche porteé (14).

13. Prothèse d'articulation suivant l'une quelconque des revendications 1 à 12, caractérisée en ce que chacun des pions (20A, 20B) est massif et forme par lui-même la calotte (17A, 17B) qu'il porte.

14. Prothèse d'articulation suivant l'une quelconque des revendications 1 à 12, caractérisée en ce que, à l'extrémité de chacun des pions (20A, 20B), est enchâssée une pièce (50) formant la calotte (17A, 17B) correspondante.

15. Prothèse d'articulation suivant l'une quelconque des revendications 1 à 14, caractérisée en ce que, à l'une au moins des broches (13, 14), est associé un fourreau (15, 16), dans lequel elle engagée par une queue (23, 30) et par rapport auquel elle peut jouer longitudinalement.

16. Prothèse d'articulation suivant la revendication 15, caractérisée en ce que, extérieurement, la broche (13, 14) concernée a un contour pyramidal, et le fourreau (15, 16) correspondant a intérieurement un contour pyramidal complémentaire du précédent.

0 278 184

*FIG.1*

*FIG.2*

*FIG.3*

*FIG.4*

*FIG.5*

*FIG.6A*

*FIG.6B*

*FIG.13*

*FIG.14*

FIG.7

FIG.8

FIG.9

FIG.10

FIG.11

FIG.12

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int Ci 4) |
|---|---|---|---|
| A,D | FR-A-2 356 406  (HAGERT)<br><br>* Figures 18-21 *<br><br>--- | | A 61 F   2/42 |
| A,D | US-A-4 059 854  (LAURE)<br><br>* Figures 1-3 *<br><br>--- | | |
| A,D | FR-A-1 484 143  (STEFFEE)<br><br>* Page 5, colonne de gauche, alinéa 2 *<br><br>--- | | |
| A | FR-A-2 321 871  (STEFFEE)<br><br>--- | | |
| E | FR-A-2 590 794  (HERMANN et al.)<br><br>* En entier *<br><br>----- | | **DOMAINES TECHNIQUES RECHERCHES (Int Ci 4)**<br><br>A 61 F |

Le present rapport de recherche a ete etabli pour toutes les revendications

| Lieu de la recherche<br>LA HAYE | Date d'achevement de la recherche<br>24-09-1987 | Examinateur<br>FISCHER G.H. |
|---|---|---|

OEB Form 1503 03 82